# FASCICULE DE BREVET EUROPEEN

(11) **EP 0 696 191 B1**
(45) Date de publication et mention de la délivrance du brevet: **22.01.1997**
(21) Numéro de dépôt: 94913676.6
(22) Date de dépôt: 21.04.1994
(51) Int. Cl.: A61K 9/16

(54) **VECTEUR PARTICULAIRE ET COMPOSITION PHARMACEUTIQUE LE CONTENANT**
PARTIKELFÖRMIGES VEKTOR UND PHARMAZEUTISCHE ZUSAMMENSETZUNG DIE DIESE ENTHÄLT
PARTICULATE VECTOR AND PHARMACEUTICAL COMPOSITION CONTAINING SUCH VECTOR

(30) Priorité: 21.04.1993 FR 9304698
(43) Date de publication de la demande: 14.02.1996
(73) Titulaire: INSTITUT PASTEUR, F-75015 Paris (FR); BIOVECTOR THERAPEUTICS SA, F-31520 Ramonville-Saint-Agne (FR)
(72) Inventeur: PERRIN, Pierre, F-77760 Villiers-Sous-Grez (FR); SAMAIN, Daniel, 31400 Toulouse (FR); CASTIGNOLLES, Nathalie, F-75015 Paris (FR)
(74) Mandataire: Texier, Christian
(86) Numéro de dépôt international: FR9400452
(87) Numéro de publication internationale: WO9423701

(56) Documents cités:
- EP-A- 0 344 040
- WO-A-90/09798
- WO-A-92/21329

## Description

La présente invention se rapporte à un nouveau type de vecteur particulaire pour le transport et la présentation de molécules de principes actifs, au moins partiellement hydrophobes, qui interagissent avec des récepteurs cellulaires spécifiques. Elle se rapporte également à un procédé d'amplification et de contrôle de l'activité de telles molécules, en particulier de molécules de lymphokines.

Les cytokines (aussi appelées lymphokines) constituent un groupe de protéines jouant un rôle important dans la régulation et le fonctionnement du système immunitaire. Elles ont des actions pléïotropiques et agissent par interaction spécifique avec des récepteurs cellulaires avec une très haute affinité. Certaines sont utilisées ou proposées comme agents thérapeutiques (ex. interférons, interleukine-2...). L'une d'entre elles, l'interleukine-2 (IL-2) est particulièrement représentative de cette famille.

L'IL-2 est synthétisée majoritairement par les lymphocytes T helper (Th1) en réponse à une stimulation par l'antigène présenté par des cellules appropriées. Elle est impliquée dans le développement des réponses immunitaires spécifiques et non spécifiques : elle interagit avec différentes cellules pour les activer (lymphocytes T et tueurs naturels-NK-), pour induire leur prolifération et leur différenciation (lymphocytes T, NK et B). L'IL-2 possède des propriétés antitumorales: elle peut induire une régression de différentes tumeurs solides, de mélanomes, de leucémies ou de lymphomes etc... L'IL-2 présente aussi un intérêt en vaccinologie : elle amplifie notamment le pouvoir protecteur de certains vaccins viraux (Herpes, rage...) et bactériens (Haemophilus pleuripneumoniae).

L'IL-1 a été proposée pour protéger les cellules myéloïdes contre l'effet cytotoxique de la chimiothérapie.

Les facteurs de croissance hématopoïtéiques, du type G-CSF et GM-CSF peuvent être utilisés dans le traitement de certains types de leucopénie, notamment après une chimiothérapie.

L'administration d'interféron γ semble limiter les phénomènes d'allergie, en bloquant la production d'IL-4.

Cependant, il s'agit là de molécules pouvant intervenir dans une cascade de réactions biologiques, parfois en synergie, et susceptibles d'entraîner des effets secondaires gênants (fièvre, érythème, oedème).

En outre, leur purification est difficile et elles sont souvent produites par génie génétique, et sont donc coûteuses.

Il serait donc souhaitable de pouvoir disposer d'un système vecteur permettant de diminuer les doses administrées, par une augmentation de l'activité observée pour une même concentration de molécule active, et par prolongation de la durée d'action dans le temps.

C'est pourquoi la présente invention a pour objet un vecteur particulaire, caractérisé en ce qu'il comporte, de l'intérieur vers l'extérieur,
- un noyau hydrophile non liquide,
- une couche externe constituée de composés lipidiques, greffés sur le noyau par des liaisons covalentes.

Le noyau de ce vecteur particulaire a, de préférence, une taille comprise entre 10nm et 5 µm.

Les systèmes de transport décrits jusqu'à aujourd'hui présentent une structure mimant celle des lipoprotéines, en particulier au niveau de la surface externe. C'est le cas notamment des liposomes, qui sont constitués d'une bicouche lipidique entourant une vacuole aqueuse. C'est également le cas pour les biovecteurs supramoléculaires (BVSM) décrits dans le brevet EP 344 040, et qui comportent une couche externe de composés amphiphiles.

Cependant, ce type de structure n'est peut-être pas utilisable pour certaines classes de principes actifs, avec lesquelles des interactions indésirables risquent de se produire, conduisant à la désorganisation du vecteur et à la perte de ses propriétés. En particulier, pour l'IL-2, il n'existe pas à ce jour de système de transport satisfaisant.

C'est donc l'un des objets de la présente invention de fournir un vecteur efficace ayant une structure plus simple. De manière surprenante, on a trouvé que des particules comprenant uniquement un noyau hydrophile, de préférence constitué d'une matrice de polysaccharides ou d'oligosaccharides naturellement ou chimiquement réticulés, et d'une couche externe lipidique, permettent de fixer des principes actifs ayant une activité biologique et d'en améliorer considérablement l'efficacité.

Le noyau peut être non ionique, ou ionique.

Un noyau ionique est obtenu par greffage, sur la matrice hydrophile, de ligands portant des fonctions pouvant être chargées positivement ou négativement. Ces fonctions peuvent être choisies dans le groupe comprenant : phosphate, sulfate, acide carboxylique, ammonium quaternaire, amine secondaire ou amine primaire.

Le greffage ionique ne doit pas être préjudiciable à la stabilité ni à la taille de la matrice polysaccharidique. Ces ligands seront donc choisis de préférence parmi des molécules biologiques naturellement présentes dans l'organisme, et couplés à la matrice par des liaisons hydrolysables enzymatiquement.

Quand les ligands greffés sur la matrice sont des composés acides, ils peuvent notamment être choisis parmi l'acide succinique, l'acide phosphorique, l'acide citrique, la glycine, l'alanine, l'acide glutamique, l'acide aspartique.

L'acide succinique est un constituant naturel de l'organisme et intervient dans le cycle de Krebs. C'est un diacide carboxylique qui peut former avec les hydroxyles de la matrice polysaccharidique une liaison ester facilement biodégradable grâce à la présence ubiquitaire dans l'organisme de succinylestérase. La fonction acide qui n'est pas engagée dans la fonction ester avec la matrice polysaccharidique reste donc disponible pour assurer les propriétés d'échange d'ions.

On peut également greffer sur la matrice des mono hydroxy ou des monoaminoacides, par exemple l'acide citrique, les acides aminés à chaîne courte et les acides aminés acides.

Selon un autre aspect de l'invention, les ligands greffés sur la matrice sont des composés basiques, fixés à la matrice par l'intermédiaire d'un composé acide.

Afin d'obtenir le greffage biodégradable de ligands basiques, il est possible d'utiliser notamment l'acide succinique entre la matrice polysaccharidique et un composé basique. L'une des fonctions acides de l'acide succinique est utilisée pour réaliser une fonction ester biodégradable avec la matrice polysaccharidique et l'autre fonction acide est utilisée pour réaliser une fonction ester ou amide avec le composé basique.

De préférence, les ligands basiques greffés sur la matrice sont des composés bifonctionnels comportant une fonction acylable formant une liaison amide ou ester avec un composé acide fixé sur la matrice.

La fonction acylable est par exemple une fonction hydroxyle ou une amine primaire.

L'autre fonction, non acylée, est par exemple une fonction amine primaire, secondaire, tertiaire, ou un ammonium quaternaire.

Le ligand peut notamment être choisi parmi, la choline, l'hydroxycholine, le 2-(diméthylamino) éthanol et la 2-(diméthylamino)éthylamine.

Dans un mode de réalisation préféré, la couche lipidique externe est constituée d'acides gras naturels. Ces acides gras naturels sont fixés uniquement à la surface du noyau par un procédé d'acylation régio-sélective qui évite la dérivation dans la masse. Ceci est obtenu en effectuant les réactions de greffage dans un milieu non solvant aprotique des polysaccharides, qui préserve la structure hydrophile particulaire du noyau et assure une répartition homogène des constituants lipidiques. La quantité de réactif lipidique, par exemple de chlorure d'acides gras pour se placer en conditions de saturation est donc relativement faible.

Le taux d'acylation du vecteur peut ainsi être modulé en réalisant un ou plusieurs cycles successifs d'acylation. Le degré d'acylation du noyau joue un rôle dans les propriétés du vecteur et sa capacité de fixation, de libération et d'amplification de l'activité des principes actifs.

La Demanderesse a montré que, si le noyau polysaccharidique seul peut fixer par exemple de l'interleukine-2, on n'observe pratiquement aucune activité biologique lorsque ces noyaux chargés en IL-2 sont placés dans un milieu cellulaire approprié. En revanche, une amplification maximale de l'activité de l'IL-2 est observée pour des vecteurs particulaires comportant un noyau avec un faible taux d'acylation.

L'augmentation du taux d'acylation diminue légèrement le phénomène d'amplification de l'activité mais prolonge la durée de cette activité.

Le vecteur particulaire selon l'invention peut de manière avantageuse être lyophilisé. La lyophilisation est effectuée dans un environnement hydrophile, qui modifie les propriétés de surface du vecteur. L'une des hypothèses, par laquelle on n'entend nullement limiter l'invention, est que lors de la lyophilisation un repliement des chaînes d'acides gras s'effectue vers l'intérieur de la structure pour laisser une surface externe hydrophile. Lors de l'utilisation, le vecteur est réhydraté directement avec la solution du principe actif à transporter.

L'invention a donc également pour objet un vecteur particulaire qui comporte en outre des molécules d'au moins un médiateur cellulaire présentant à la fois un caractère hydrophobe et un caractère ionique, du type agissant par l'intermédiaire d'un récepteur membranaire ; lesdites molécules sont associées par des interactions hydrophobes à la couche externe de composés lipidiques du vecteur, et/ou par des interactions ioniques au noyau.

Ces médiateurs cellulaires peuvent être des polypeptides, glycosylés ou non, qui jouent un rôle dans le fonctionnement du système immunitaire.

Dans tous les cas, les molécules de principe actif associées à la couche lipidique externe doivent présenter un caractère partiellement hydrophobe. La Demanderesse a en effet mis en évidence que les interactions entre le vecteur selon l'invention et un médiateur cellulaire comme l'IL-2 sont rompues par l'addition d'un détergent non ionique, du type Hecameg (6-0-(N-heptylcarbamoyl)méthyl-α-D-glucopyranoside). Il se produit en outre des interactions ioniques entre le noyau, qui présente un caractère acide (notamment grâce au greffage de radicaux phosphates) et les fonctions basiques de l'IL-2.

Parmi les molécules de médiateurs cellulaires pour lesquelles le vecteur particulaire présente un intérêt particulier, il faut citer les cytokines qui regroupent un ensemble de médiateurs solubles produits par les cellules. Elles comprennent les lymphokines ou interleukines, secrétées par des cellules immunocompétentes qui assurent les interactions entre les leucocytes, les monokines, les TNF (ou tumor necrosis factor α et β ), les différents interferons (INF α, β et γ), le G-CSF, le M-CSF, le GM-CSF et le MIF.

Parmi les médiateurs les mieux adaptés, l'IL-2 voit son activité améliorée par l'association aux vecteurs particulaires selon l'invention ; l'IL-1, qui possède une forme membranaire de 23 kD et la forme membranaire de 26 kD du TNF sont particulièrement appropriés à la préparation de vecteurs selon l'invention.

Le taux d'acylation varie afin d'obtenir la meilleure interaction avec les molécules de médiateurs associées, qui dépend des rapports respectifs hydrophilie/hydrophobie de la couronne lipidique et du principe actif, compte tenu de l'effet, d'amplification ou de retard dans la libération, recherché.

L'invention a également pour objet des compositions pharmaceutiques contenant des vecteurs particulaires selon l'invention, chargés ou non par des molécules de médiateur cellulaire, et un véhicule pharmaceutiquement acceptable.

Les applications des vecteurs et des compositions selon l'invention comprennent la préparation de formes pour administration in vivo de principe actif. Elles comprennent également la supplémentation de milieux de culture in vitro de lignéees cellulaires, produisant par exemple une protéine d'intérêt.

De manière générale, l'un des objets de l'invention est un procédé d'amplification de l'activité d'un médiateur cellulaire polypeptidique au moins partiellement hydrophobe, caractérisé en ce que
a) on prépare un vecteur particulaire qui comporte de l'intérieur vers l'extérieur
   - un noyau constitué d'une matrice de polysaccharides ou d'oligosaccharides naturellement ou chimiquement réticulés,
   - une couche externe constituée d'acides gras naturels greffés au noyau par des liaisons covalentes, à un taux variable,
b) de manière facultative, on lyophilise le vecteur obtenu dans un environnement hydrophile.
c) on associe le médiateur cellulaire au vecteur par des interactions au moins partiellement hydrophobes.

L'effet sur l'activité du médiateur cellulaire peut notamment comprendre une augmentation de l'activité intrinsèque et/ou une augmentation de la durée de vie du principe actif associé au vecteur selon l'invention.

Selon l'un des aspects de l'invention, les vecteurs, constitués du noyau hydrophile réticulé, portant de préférence des ligands ioniques, sur lequel sont fixés les résidus d'acides gras, appelés aussi "coeurs acylés", sont lyophilisés. Le chargement en principe actif protéique s'effectue par mise en présence des coeurs acylés lyophilisés avec une quantité limitée de solution aqueuse protéique de principe actif, de manière à assurer la formation d'agrégats stables de vecteurs chargés en principe actif. Ces agrégats sont ensuite dispersés dans une phase aqueuse par dilution.

D'autres caractéristiques et avantages de la présente invention apparaîtront à la lecture des exemples qui suivent.

Dans ces exemples, on se référera aux figures suivantes :
- **Figure 1 :**: Prolifération des cellules CTLL en présence d'IL-2 mélangée aux noyaux polysaccharidiques acylés (lot 14) : nombre de cellules vivantes (comptage MTT).
- **Figure 2 :**: Prolifération des cellules CTLL en présence d'IL-2 mélangée aux noyaux polysaccharidiques acylés : capacité proliférative des cellules.
- **Figure 3 :**: Influence de l'association de l'IL-2 à des noyaux polysaccharidiques acylés sur l'activité proliférative des cellules l'IL-2 dépendantes.
- **Figure 4 :**: Influence de la présence de résidus acyl (CA lot 18) sur les noyaux polysaccharidiques (NPS) sur l'amplification des propriétés de l'IL-2.
- **Figure 5 :**: Croissance des cellules CTLL en présence de deux quantités d'IL-2 (4 et 20 U).
- **Figure 6a :**: Influence du nombre d'acylations des noyaux sur leur capacité à amplifier les propriétés biologiques de l'IL-2 : croissance des cellules IL-2 dépendantes en présence de 20 U d'IL-2 mélangés à des noyaux différemment acylés.
- **Figure 6b :**: Influence du nombre d'acylations des noyaux sur leur capacité à amplifier les propriétés biologiques de l'IL-2 : capacité proliférative des cellules IL-2 dépendantes.
- **Figure 7a :**: Influence du nombre d'acylations des noyaux sur leur capacité à amplifier les propriétés biologiques de l'IL-2 : croissance des cellules IL-2 dépendantes en présence de 4 U d'IL-2 mélangés à des noyaux différemment acylés..
- **Figure 7b :**: Influence du nombre d'acylations des noyaux sur leur capacité à amplifier les propriétés biologiques de l'IL-2 : capacité proliférative des cellules IL-2 dépendantes.
- **Figure 8a :**: Influence de la quantité de noyaux sur les propriétés biologiques de l'IL-2 : croissance des cellules IL-2 dépendantes en présence de 0,1 mg et de 2 mg de noyaux acylés.
- **Figure 8b :**: Influence de la quantité de noyaux sur les propriétés biologiques de l'IL-2 : capacité proliférative des cellules IL-2 dépendantes.
- **Figure 9 :**: Influence de la présence de résidus acyl (CA lot 18) sur les noyaux polysaccharidiques (NPS) sur l'amplification des propriétés de l'IL-2.
- **Figure 10 :**: Influence du nombre d'acylations des NPS sur la stabilité et/ou la libération d'IL-2 dans le milieu de culture : dosage de l'IL-2 résiduelle dans les surnageants de culture.
- **Figure 11 :**: IL-2 résiduelle dans les surnageants de culture.
- **Figure 12 :**: Influence de la quantité de noyaux polysaccharidiques acylés sur la quantité d'IL-2 résiduelle dosée dans les surnageants de culture des cellules CTLL.
- **Figure 13 :**: Croissance des cellules IL-2 dépendantes en présence d'IL-2 liée aux noyaux acylés.
- **Figure 14 :**: Effet des noyaux acylés sur la restauration de l'activité sur la prolifération cellulaire de l'IL-2 dénaturée. La ligne continue et les carrés représentent l'activité de l'IL-2 dénaturée, les cercles l'activité de l'IL-2 dénaturée associée aux noyaux CA0, les triangles l'activité de l'IL-2 dénaturée associée aux noyaux CA1, et la ligne pointillée représente l'activité d'IL-2 native, sur 2.10⁴ cellules CTLL.

### Exemple 1 : Préparation de particules chargées en IL-2

### Interleukine-2 :

- recombinante humaine (Genzyme),
- recombinante humaine radiomarquée (³⁵S Méthionine et cystéine) préparée à partir de cellules BHK-21 recombinée,
- recombinante souris (Genzyme).

### Synthèse des noyaux polysaccharidiques (NPS)

300 g d'amidon Waxylis 100 (Roquette, France) sont dissouts dans de la soude 2 N puis 8 ml d'épichlorhydrine sont ajoutés. On obtient un gel qui est neutralisé à l'acide acétique, puis lavé à l'eau. Ce gel est dilué dans 2,25 1 de soude 1,5 N, puis 57 ml de POCl₃ à un débit de 9 ml/heure et 342 ml de soude 10 N à un débit de 54 ml/heure sont ajoutés. Le gel est neutralisé à l'acide acétique et broyé avec un homogénéisateur haute pression Minilab. La suspension obtenue est centrifugée 15 minutes à 10 000 g, l'analyse du surnageant au nanosizer montre une population homogène de particules de 200 nm. Ces NPS sont lyophilisés en présence d'hydrogénocarbonate d'ammonium. On désigne par CAO les noyaux polysaccharidiques non acylés.

### Synthèse des coeurs acylés (CA1, CA2 et CA3)

30 g de NPS lyophilisés sont mélangés à 450 ml de dichlorométhane fraichement distillé. On ajoute 3,3 ml de chlorure d'acide palmitique sous agitation. Après 2 heures de réaction, le milieu réactionnel est filtré et lavé à l'éther éthylique. Les CA1 sont ensuite ultrafiltrés puis lyophilisés. Les coeurs acylés obtenus peuvent suivre 1 à 2 cycles d'acylation identiques à celui décrit, ceci améliorant l'homogénéité de la couronne d'acides gras à l'extérieur des particules. La taille des particules acylées reste constante à 200 nm. Nous obtenons après un cycle d'acylation des CA1, 2 cycles des CA2, 3 cycles des CA3. Ces coeurs acylés sont lyophilisés et utilisés sous forme lyophilisée.

### Association de l'IL-2 aux CA

Suivant les expériences, des quantités variables d'IL-2 peuvent être associées aux noyaux polysaccharidiques acylés et non acylés. Pour 10 unités d'IL-2/mg de CA, on hydrate O,5 mg de CA directement avec 200 µl d'IL-2 préalablement diluée dans du tampon PBS (50 unités/ml). Après agitation douce et un repos d'une heure à 4° C, la suspension est additionnée de 1 ml de milieu de culture des cellules.

Selon les besoins, le mélange est testé immédiatement ou purifié pour séparer l'IL-2 qui n'a pas été associée aux CA.

### Séparation de l'IL-2 qui n'a pas été associée aux CA

Le mélange IL-2/CA est centrifugé (5 min à 2000 g ). Le surnageant (S) est délicatement prélevé pour être testé et le culot (C : IL-2 liée) est lavé deux fois avec du milieu puis resuspendu dans le milieu de culture supplémenté.

### Mise en évidence de l'association de l'IL-2 aux noyaux polysaccharidiques acylés

L'association de l'IL-2 aux noyaux polysaccharidiques et aux noyaux polysaccharidiques acylés est possible comme le montrent les résultats reportés dans le tableau 1 ci-dessous ; en effet, puisqu'on enregistre une nette diminution de la concentration de l'IL-2 contenue dans le surnageant. Toutefois, ce n'est qu'avec les noyaux polysaccharidiques acylés que l'IL-2 n'a pu être libérée après la reprise du culot contenant l'IL-2 fixée.

**Tableau 1**

| **Interaction entre l'Interleukine-2 recombinante humaine et les noyaux polysaccharidiques: importance de l'acylation.** | | |
|---|---|---|
| Nature du produit | Activité biologique de l'IL-2 (Unités par ml) Après association avec : | |
| | Noyaux polysaccharidiques | Noyaux polysaccharidiques acylés |
| Mélange* | 1,1 | 5 |
| Surnageant* | 0,3 | 0,4 |
| Culot + PBS* | 0,5 | 2,7 |
| IL-2 témoin : 1,4 Unités par ml L'IL-2 recombinante humaine a été mélangée soit aux noyaux polysaccharidiques soit aux noyaux polysaccharidiques acylés et centrifugée pour partie du mélange après intéraction le jour même. Après 7 jours de conservation a 4° C, la quantité d'IL-2 présente dans chaque type de produit a été déterminée selon la méthode de Gillis ( J. Immunol. 1978, **120**, 2027). | | |

| | | |
|---|---|---|
| * Mélange : mélange IL-2/noyaux conservé 7 jours a 4° C. | | |
| * Surnageant : IL-2 non associée aux noyaux et conservé 7 jours à 4° C. | | |
| * Culot + PBS : IL-2 associée aux noyaux et libérée pendant les 7 jours de conservation. | | |

L'association de l'IL-2 aux noyaux polysaccharidiques est plus importante lorsqu'ils ont été acylés. Ceci est mis en évidence par le dosage de l'activité biologique de l'IL-2 contenue dans le surnageant d'interaction (5 heures) après élimination des coeurs acylés (CA). Ces résultats sont présentés dans le tableau 2.

**Tableau 2**

| |
|---|
| Quantité d'IL-2 initiale : 70 U |

| Contenu en IL-2 dans le surnageant d'interaction avec : |
|---|
| - CAO : 30,0 U |
| - CA1 : 0,2 U |
| - CA2 : 0,2 U |
| - CA3 : 0,2 U |
| avec CA0, 50% de l'IL-2 a été associée au CA ou inactivée tandis qu'avec les CA acylés toute l'IL-2 a été associée au CA ou inactivée. Le nombre d'acylations ne semble pas ici influencer sur la capture de l'IL-2. Le même type de résultat est obtenu avec l'IL-2 radiomarquée (Tableau 3). |

**Tableau 3**

| **Mise en évidence d'une association entre l'IL-2 radiomarquée et les noyaux polysaccharidiques acylés ou non.** | | | | |
|---|---|---|---|---|
| Nature des noyaux polysaccharidiques | IL-2 non associée aux noyaux* | Moment de contrôle de la libération de l'IL-2* associée aux noyaux | | |
| | | 12 H | 24 H | 96 H |
| CAO | 540 | 27 | 23 | 15 |
| CA1 | 160 | 41 | 28 | 13 |
| CA2 | 220 | 46 | 24 | 20 |
| CA3 | 130 | 36 | 24 | 20 |

| | | | | |
|---|---|---|---|---|
| * Les résultats représentent le nombre cpm pour 20 µl. | | | | |

De l'IL-2 radiomarquée (1700 cpm/20 µl) a été utilisée pour hydrater les noyaux. Après 5 heures d'interaction, le mélange est centrifugé et le surnageant est contrôlé (IL-2 non associée). Le culot de centrifugation est ensuite remis en suspension dans du PBS pendant 7 heures puis centrifugé et contrôlé (point 12 H). Le nouveau culot est à nouveau remis en suspension dans du PBS pendant 12 heures (point 24 H) et ainsi de suite.

On constate que là aussi l'acylation induit une association plus importante de l'IL-2. Il apparait que l'IL-2 associée aux noyaux n'est que très faiblement libérée au cours du temps en présence de tampon. Toutefois, malgré la faiblesse de la radioactivité, il semble que la libération de l'IL-2 soit plus importante à partir des structures acylées.

### Exemple 2 : Nature de l'interaction de l'IL-2 avec les noyaux

Afin de déterminer la nature de l'interaction entre l'IL-2 et les noyaux, l'IL-2 liée aux noyaux a été éluée avec différentes solutions : tampon PBS, solution de détergent (Hecameg 1%) ou de NaCl 0,5 M. Comme le montrent les résultats préliminaires reportés dans le tableau 4, pas d'IL-2 est libérée avec le PBS tandis qu'une quantité importante est libérée avec le détergent et très peu ou pas avec l'élévation de la force ionique. Il apparaît donc que la nature de l'interaction entre l'IL-2 qui a été libérée et les noyaux soit plutôt hydrophobe qu'hydrophile, y compris avec les noyaux qui n'ont pas été acylés.

**Tableau 4**

| **Mise en évidence d'une association entre l'IL-2 et les noyaux polysaccharidiques acylés ou non : IL-2 (activité biologique) libérée (12 heures d'interaction) à partir des CA débarrassés de l'IL-2 initiale.** | | | |
|---|---|---|---|
| Nature des noyaux polysaccharidiques | Libération d'IL-2* Nature du traitement des noyaux après interaction avec l'IL-2 | | |
| | PBS | Hecameg 1% | NaCl 0,5 M |
| CA0 | 0 | + | +/- |
| CA1 | 0 | + | +/- |
| CA2 | 0 | + | + |
| CA3 | 0 | + | 0 |

| | | | |
|---|---|---|---|
| * Les résultats représentent une lecture optique de la prolifération des cellules CTLL. | | | |

Les interactions entre l'IL-2 et les noyaux acylés sont très fortes puisque peu d'IL-2 est libérée au cours de 23 jours de conservation a 4° C. Par contre, après ce temps de conservation une quantité importante d'IL-2 peut être libérée par un détergent/hecameg 1%).

**Tableau 5**

| **Mise en évidence d'une association entre l'IL-2 radiomarquée et les noyaux : étude du type d'interaction majeure entre l'IL-2 et les noyaux.** | | | |
|---|---|---|---|
| Nature des noyaux polysaccharidiques | Libération de l'IL-2 (cpm pour 20 µl) à partir des noyaux sédimentés après 23 jours et après traitement par : | | |
| | Aucun surnageant | PBS | Hecameg |
| CAO | 22 | 31 | 32 |
| CA1 | 28 | 25 | 283 |
| CA2 | 52 | 44 | 213 |
| CA3 | 72 | 52 | 329 |

### Exemple 3 : Evaluation de l'effet de l'association de l'IL-2 aux CA

### a) Matériel et méthodes

On utilise comme cellules indicatrices des cellules murines CTLL (cytotoxic T lymphocyte line) dont la survie et la croissance dépendent de la présence et la quantité d'IL-2. Elles sont cultivées en milieu RPMI supplémenté (glutamine, antibiotiques, sérum foetal bovin, sodium pyruvate, acides aminés non essentiels, Hepes, et β-mercaptoethanol).

Dans des plaques 24 puits les échantillons (1 ml) sont testés en les ajoutant à 1 ml de cellules CTLL, à 2.10⁵ cellules par ml. Les plaques sont ensuite incubées à 37° C sous atmosphère humide et en présence de 7% de CO₂. Chaque jour du contrôle l'ensemble du puits à tester est prélevé et centrifugé. Le surnageant est récupéré et congelé pour mesurer ultérieurement l'IL-2 (IL-2 résiduelle). Le culot cellulaire est resuspendu dans du milieu de culture pour un dénombrement des cellules vivantes.

La croissance des cellules est aussi testée d'après leur capacité à incorporer de la thymidine tritiée dans leur génome au jour du contrôle. A cette fin, dans des plaques 96 puits, 100 µl d'échantillon sont mélangés à 100 µl de cellules contenant 2.10⁵ cellules par ml. Le jour du contrôle de la thymidine tritiée est ajoutée pendant 5 heures et la radioactivité est déterminée après lyse et filtration des cellules.

### Méthodes de contrôle

### - Dénombrement des cellules vivantes

Le dénombrement des cellules vivantes est effectué d'après le taux d'incorporation d'un colorant vital (MTT) dans les cellules en culture.

### - Titrage de l'IL-2

L'IL-2 contenue dans les différents produits d'association avec les noyaux acylés ou l'IL-2 résiduelle est dosée suivant la technique usuelle (Méthode de Gillis: prolifération).

### b) Conséquence de l'association de l'IL-2 à des noyaux acylés sur la prolifération des cellules IL-2 dépendantes

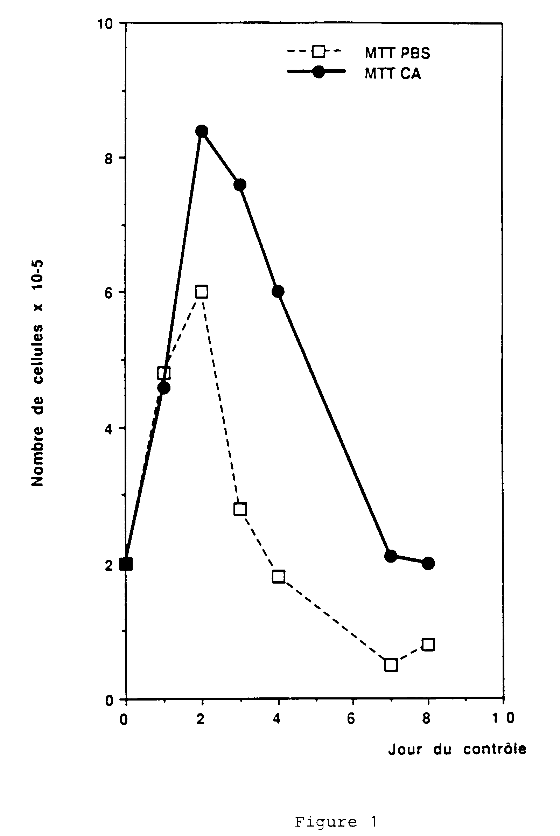

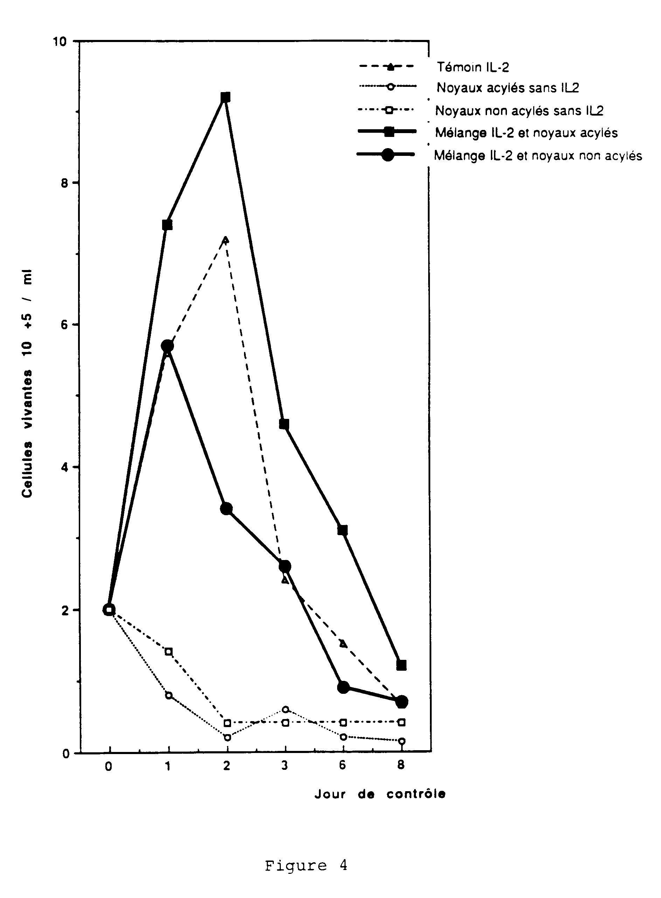

Lorsque le mélange IL-2-noyaux polysaccharidiques acylés (Lot 14) est ajouté dans le milieu de culture des cellules CTLL, on constate (Fig. 1) que la quantité de cellules qui ont proliféré est plus élevée qu'en présence d'une même concentration d'IL-2 seule, sous forme diluée dans du PBS. Le même type de résultats est obtenu avec un autre lot de noyaux polysaccharidiques acylés (Lot 18: Fig. 4).

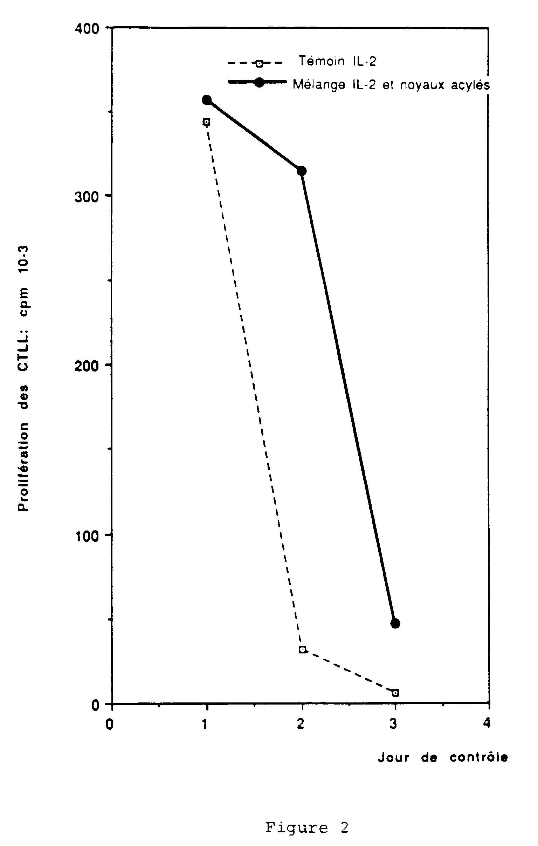

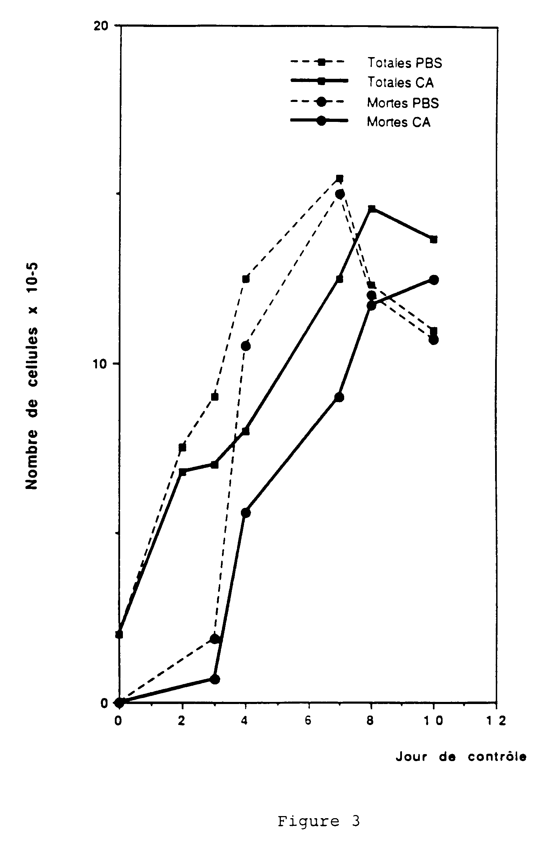

De plus, si au lieu de dénombrer les cellules vivantes, on évalue la capacité des cellules à proliférer dans la culture (incorporation de thymidine tritiée) (Fig. 2), la présence du mélange d'Il-2 et de noyaux polysaccharidiques acylés augmente significativement cette capacité. Non seulement de l'IL-2 encore active sur de nouvelles cellules est présente dans les surnageants mais cette IL-2 mélangée aux noyaux polysaccharidiques acylés entraîne une prolongation de la prolifération des cellules. Cette présence du mélange a deux conséquences (Fig. 3): 1°) tout au long de la culture (sauf au jour 10) le nombre de cellules mortes est moindre en présence de noyaux polysaccharidiques acylés ; 2°) le nombre total de cellules en fin de culture est plus important en présence de noyaux polysaccharidiques acylés.

### c) Influence de l'acylation des noyaux polysaccharidiques sur leur capacité à amplifier les propriétés de l'IL-2

En l'absence d'IL-2, les noyaux polysaccharidiques (NPS) aussi bien que les noyaux polysaccharidiques acylés (CA) ne sont pas capables d'induire ni la croissance ni la survie des cellules (Fig. 4). Par contre, les noyaux polysaccharidiques acylés amplifient les propriétés de l'IL-2. D'autre part, les noyaux polysaccharidiques non acylés ont plutôt un effet négatif sur la croissance des cellules (Fig. 4).

### Exemple 4 : Influence du degré d'acylation et de la quantité du mélange IL-2/noyaux sur l'amplification des propriétés de l'IL-2

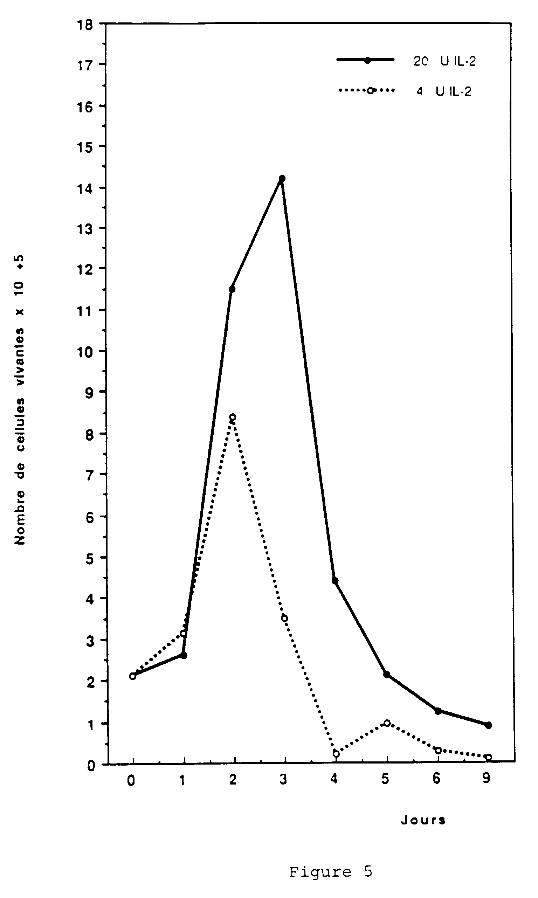

Dans une série d'expériences, deux quantités du mélange IL-2/noyaux ont été testées : 20 unités d'IL-2 ont été associées aux noyaux et éventuellement diluées au 1/5 (la quantité de noyaux en a été tout autant diluée). Il est couramment vérifié que lorsque des quantités importantes d'IL-2 sont ajoutées aux cellules indicatrices une inhibition de la croissance des cellules en résulte. Nous avons vérifié que les conditions initiales d'interaction entre l'IL-2 et les noyaux ne relevaient pas de ce cas (Fig. 5) : avec 4 unités les cellules prolifèrent moins qu'avec 20 unités.

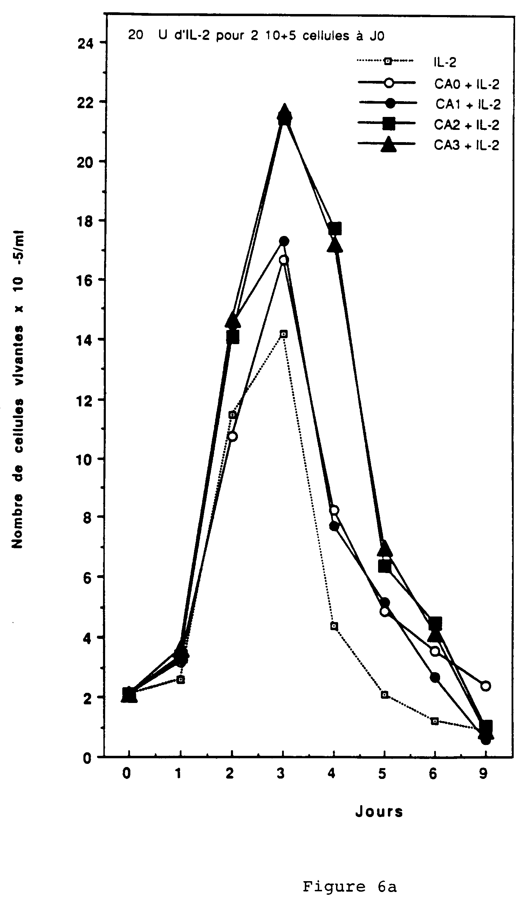

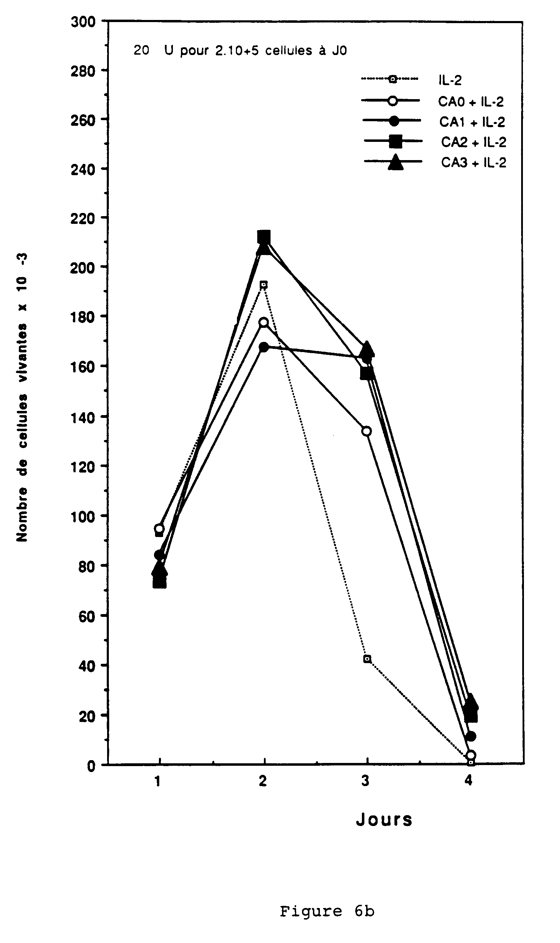

Avec 20 unités d'IL-2, on constate que la croissance des cellules (Fig. 6a) avec l'IL-2 mélangée aux noyaux est principalement augmentée avec les noyaux ayant subi 2 ou 3 acylations. Le même type de résultats mais moins prononcé est obtenu en mesurant la capacité des cellules à proliférer (Fig. 6b).

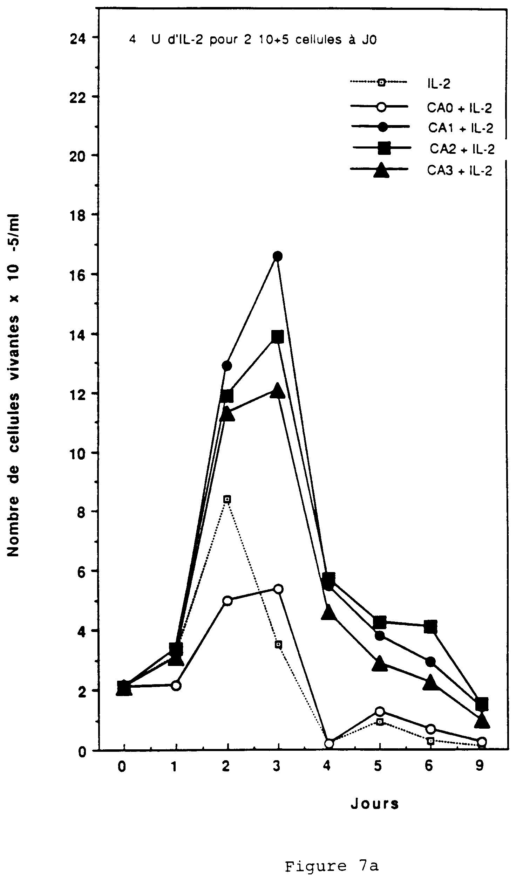

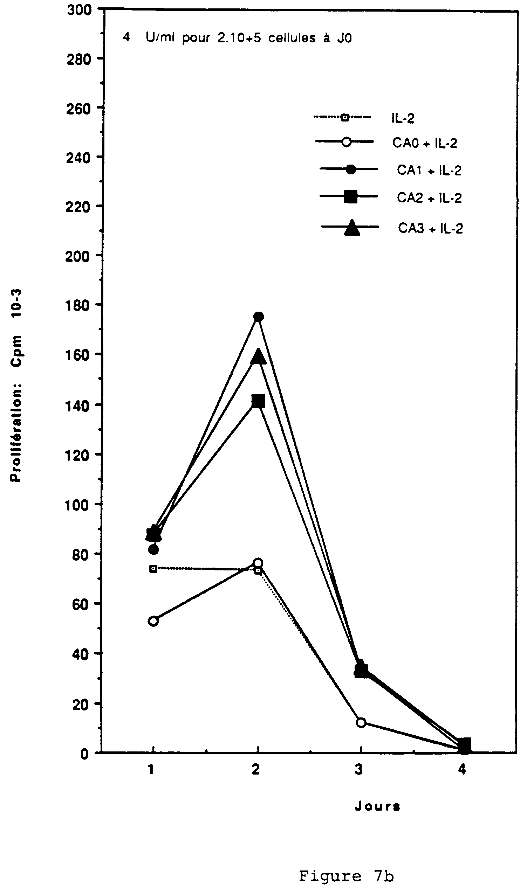

Avec 4 unités d'IL-2, les résultats (Fig. 7a et 7b) sont plus tranchés : l'acylation joue un rôle important comme déjà démontré (Fig. 4). Toutefois, une augmentation de l'acylation diminue l'amplification de la croissance (Fig. 7a) aussi bien que la capacité proliférative des cellules (Fig. 7b).

Il est possible que le système soit inhibé par excès d'activité de l'IL-2, pour 20 U d'IL-2.

### Influence de la quantité de noyaux acylés sur l'amplification des propriétés de l'IL-2

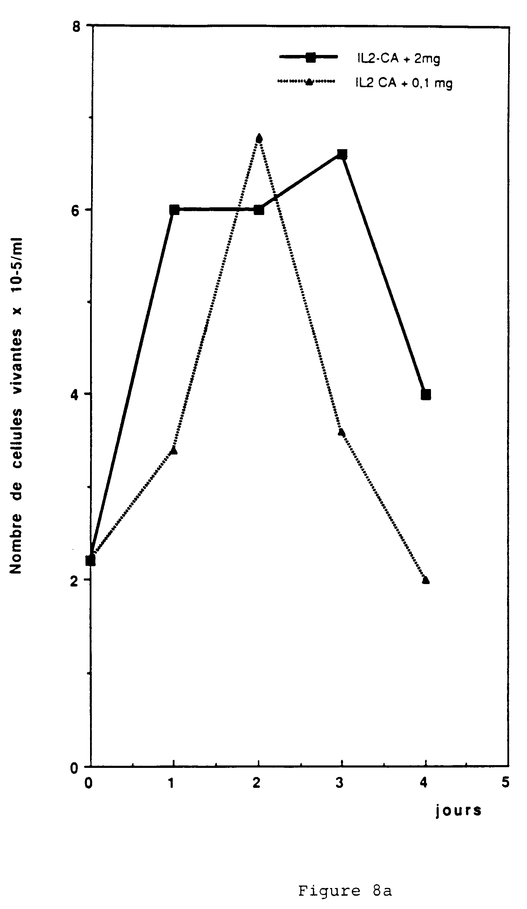

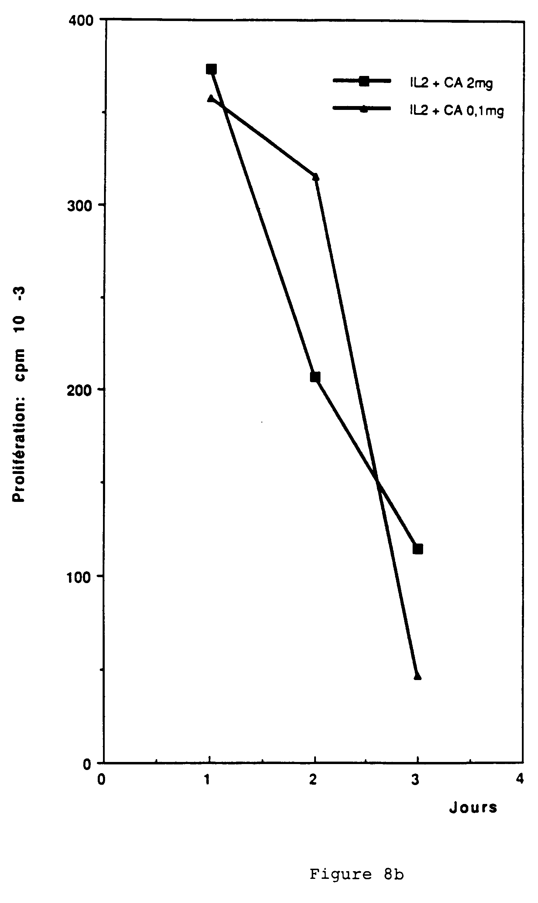

Une même quantité d'IL-2 a été mélangée soit à 2 mg soit à 0,1 mg de noyaux acylés pour étudier l'influence de la quantité de noyaux sur l'amplification des propriétés de l'IL-2. Comme le montrent les résultats reportés dans la figure 8, c'est surtout aux jours 1 et 3 qu'apparaissent les différences : la croissance (Fig. 8a) et la capacité proliférative (Fig. 8b) sont plus importantes pour la quantité la plus élevée de noyaux.

### Exemple 5 : Conséquence de l'association de l'IL-2 à des noyaux acylés ou non sur la présence d'IL-2 résiduelle dans les surnageants de culture des CTLL

Il apparait, qu'à côté d'une amplification de l'activité spécifique de l'IL-2, un phénomène de protection" de l'IL-2 ou de libération lente d'une IL-2 active pourrait être observé dans le cas où celle-ci a été préalablement associée aux noyaux polysaccharidiques acylés. Plusieurs de ces trois hypothèses pourraient même être réalisées sumultanément. Pour clarifier ce point, dans les différentes expériences de prolifération des cellules, on a titré l'IL-2 résiduelle présente à différents temps de la culture.

### a) Influence de l'acylation des noyaux polysaccharidiques sur la quantité d'IL-2 résiduelle dans les cultures

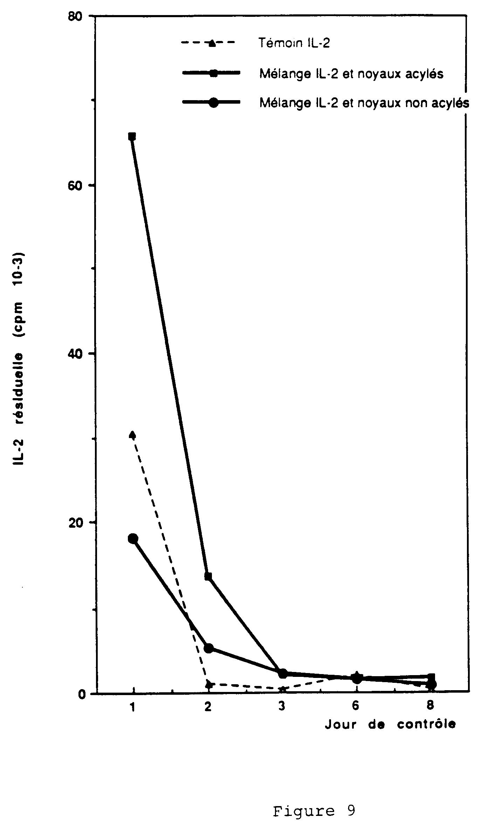

Comme le montrent les résultats reportés dans la figure 9 (mêmes conditions que la Fig. 4), d'avantage d'IL-2 est présente dans les surnageants des cultures ayant reçu le mélange d'IL-2 avec des noyaux acylés, la présence du mélange d'IL-2 et de noyaux non acylés ayant plutôt pour effet de faire diminuer l'IL-2 résiduelle. Toutefois, dès le début, la quantité d'Il-2 résiduelle apparente est nettement plus élevée que celle observée avec la préparation d'IL-2 témoin indiquant que soit l'IL-2 a été mieux protégée durant les premières 24 heures du culture soit l'activité spécifique de l'IL-2 a été amplifiée (amélioration de la présentation de l'IL-2).

### b) Influence du degré d'acylation et de la quantité du mélange IL-2/noyaux sur la quantité d'IL-2 résiduelle dans les cultures

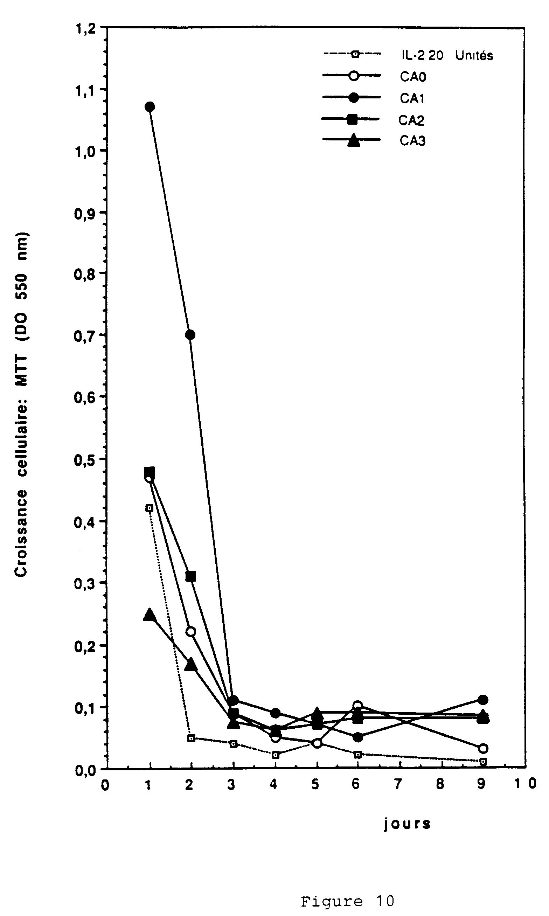

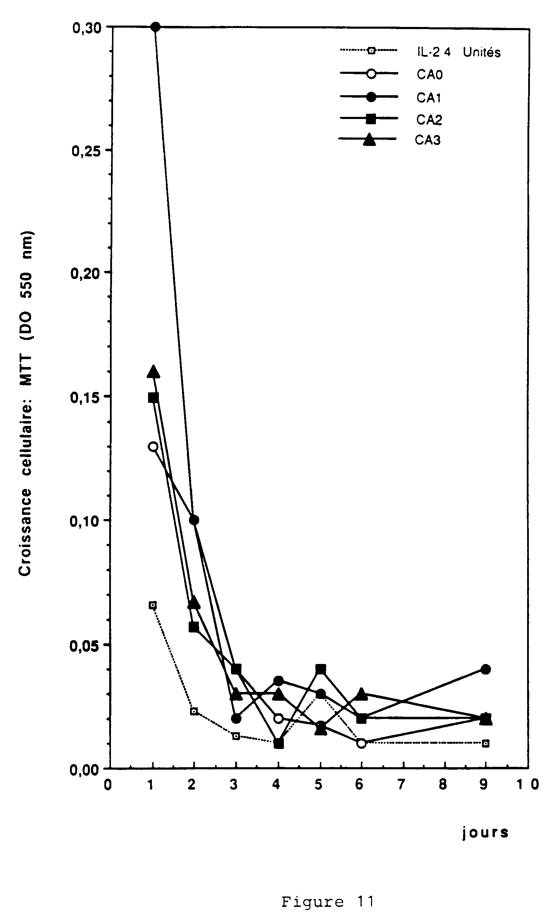

L'IL-2 résiduelle a aussi été testée dans les surnageants de culture correspondant aux figures 6 et 7. Comme précédemment, on enregistre une quantité d'IL-2 résiduelle très importante lorsque les noyaux ont subi une acylation, que ce soit avec 20 ou 4 unités d'IL-2. Tout comme pour la prolifération, l'augmentation de l'activité de l 'IL-2 est plus significative en présence d'une quantité plus faible d'IL-2 (20 U Fig. 10 et 4 U Fig. 11). Plusieurs acylations conduisent à une activité inférieure à celle obtenue avec une seule acylation.

### c) Influence de la quantité de noyaux sur la quantité d'IL-2 résiduelle dans les cultures

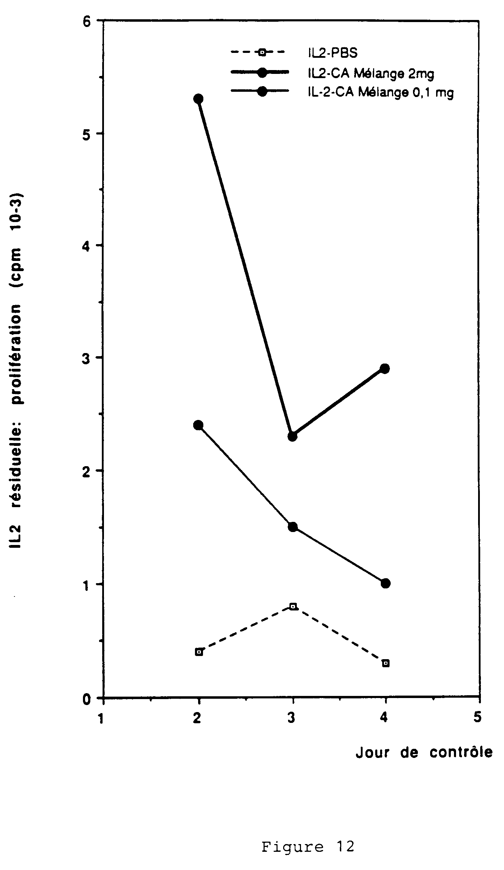

La quantité d'IL-2 résiduelle dépend aussi de la quantité de noyaux acylés auxquels elle a été associée comme le montre la figure 12. Là encore, on observe le rôle important des noyaux acylés.

### Exemple 6 : Mise en évidence de propriétés biologiques différentes entre l'IL-2 liée et l'IL-2 libre

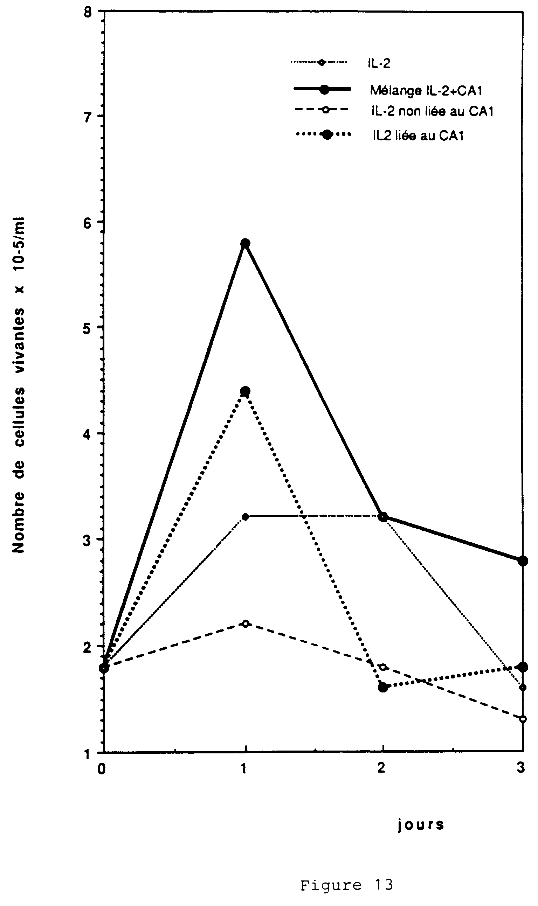

20 unités d'IL-2 sont mélangés à des noyaux acylés (CA1) puis centrifugés. Le culot et le surnageant ont été testés séparément pour leur aptitude à induire la croissance des cellules (Fig. 13). On constate comme précédemment, que le mélange IL-2/CA amplifie les propriétés de l'IL-2. Il en est de même pour l'IL-2 liée aux CA. Par contre le surnageant, ici encore, ne contient plus suffisamment d'IL-2 pour induire une prolifération.

### Exemple 7 : interaction entre l'interleukine 2 et les biovecteurs : influence de l'acylation

La possibilité d'interaction de l'IL-2 avec les noyaux acylés a été déterminée. L'IL-2 est mélangée avec des noyaux (acylés ou non), diluée dans du milieu de culture cellulaire supplémenté avec 10% de FBS (sérum foetal bovin), incubée à 37° C et testée à des temps déterminés pour son activité sur la prolifération en mélange avec les vecteurs ou après leur élimination.

Les surnageants correspondant aux mélanges IL-2/vecteurs montrent une diminution significative de l'activité sur la prolifération immédiatement après l'élimination des polymères, par rapport à l'activité de l'IL-2 seule. On ne récupère que 15% et 70% de l'activité de l'IL-2, respectivement avec les noyaux CA2 et CA0. L'IL-2 associée aux vecteurs est encore active car les culots remis en suspension peuvent induire la croissance des cellules CTLL jusqu'à 4 jours après qu'ils aient été ajoutés aux cellules CTLL. Ces données suggèrent fortement que l'IL-2 est associée aux vecteurs, car elle est éliminée au cours de la centrifugation. En outre, l'activité sur la prolifération de l'IL-2 est conservée jusqu'au jour 4, avec un maximum de deux jours après son association avec les vecteurs.

En ce qui concerne la stabilité de l'IL-2, après deux jours, l'activité sur la prolifération de l'IL-2 seule décroît très fortement alors qu'une prolifération significative peut encore être mise en évidence avec de l'IL-2 après interaction avec des noyaux CA0 ou CA1. Ceci peut suggérer que l'IL-2 associée aux vecteurs est progressivement relarguée dans les conditions de cultures cellulaires. Le relarguage de l'IL-2 est plus important pour les noyaux CAO et CA1.

L'activité sur la prolifération de l'IL-2 mélangée avec des vecteurs est aussi mesurée après stockage a 37° C. L'augmentation de l'activité de l'IL-2 est mise en évidence quand l'IL-2 a été incubée avec des vecteurs quelque soit le jour de contrôle. L'effet le plus important est observé après 24 heures avec des noyaux CA2.

Dans ces conditions, les noyaux non acylés augmentent faiblement l'activité de l'IL-2 contrairement à ce qui a été observé dans les expériences précédentes. Cette différence peut s'expliquer par une association lente de l'IL-2 avec les noyaux CA0, comparés aux noyaux acylés. Après deux jours de culture, l'activité de l'IL-2 décroît progressivement, probablement à la fois à cause de la dénaturation et de la libération.

Ces résultats montrent clairement que l'IL-2 peut être associée avec les vecteurs (de manière plus efficace lorsqu'ils sont acylés) et que l'IL-2 associée aux vecteurs a une activité sur la prolifération ; l'IL-2 associée aux vecteurs particulaires peut être relarguée sous forme active.

### Exemple 8 : rétablissement de l'activité sur la prolifération d'une IL-2 dénaturée par association avec des vecteurs particulaires selon l'invention

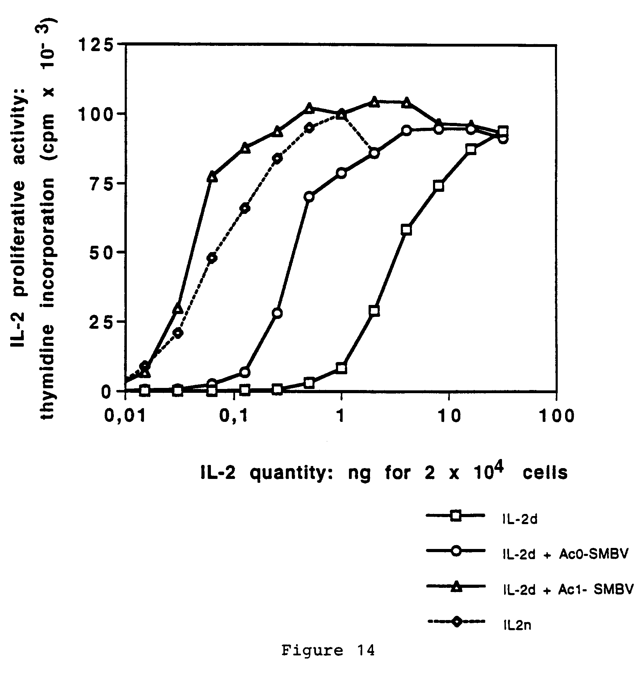

De l'IL-2 humaine recombinante purifiée produite par la levure (Genzyme Corp) est dénaturée ou agrégée par reconstitution et dilution dans du tampon PBS suivi par une congélation à - 20° C puis décongélation. Dans ces conditions, on obtient une réponse à 50% avec environ 40 ng/ml (ou 4 ng pour 2.10⁴ cellules CTLL) au lieu d'environ 0,8 ng/ml (ou 0,08 ng pour 2.10⁴ cellules CTLL) pour la préparation d'IL-2 native, comme montré sur la figure 14. Cette diminution importante de l'activité de l'IL-2 (50 à 100 fois moins) confirme que le traitement par PBS et congélation/décongélation réduit la biodisponibilité de l'IL-2.

L'IL-2 dénaturée est aussi incubée avec des noyaux acylés. Ce traitement restaure partiellement ou totalement l'activité de l'IL-2 dans cette préparation quand elle est mélangée respectivement avec des noyaux CA0 ou CA1. Après association avec les noyaux CA0 ou CA1 (ainsi qu'avec des noyaux CA2 ou CA3) environ 10 à 100 fois moins d'IL-2 est respectivement nécessaire pour induire le même niveau de prolifération qu'avec l'IL-2 seule. Après association avec des noyaux acylés, l'activité de l'IL-2 sur la prolifération est la même ou supérieure que celle avant la dénaturation. En conséquence, à côté de leur effet sur l'IL-2 non dénaturée, les vecteurs selon l'invention peuvent restaurer l'activité de l'IL-2 dénaturée sur la prolifération in vitro. Cette restauration est plus marquée lorsque les vecteurs sont acylés.

### Exemple 9 : Influence de l'origine de l'IL-2 sur la capacité des noyaux polysaccharidiques acylés à amplifier les propriétés de l'IL-2

A côté de l'IL-2 recombinante humaine (IL-2 Hr), de l'IL-2 de rat a été associée aux noyaux polysaccharidiques acylés. Le même phénomène d'amplification est observé, comme indiqué sur le tableau 6.

**Tableau 6**

| **Influence de l'origine de l'IL-2 sur la capacité des noyaux polysaccharidiques acylés à amplifier les propriétés de l'IL-2 vis-à-vis de la prolifération des cellules (contrôle au jour 3).** | | |
|---|---|---|
| Nature du produit | Prolifération des cellules (cpm 10⁻³) | |
| | Non associée | Associée aux noyaux acylés |
| IL-2 Hr | 11 | 31 |
| IL-2 rat | 17 | 83 |
| IL-2 rat 1/10 | 1 | 2 |

## Revendications

1. Vecteur particulaire, caractérisé en ce qu'il comporte, de l'intérieur vers l'extérieur,
- un noyau hydrophile non liquide,
- une couche externe constituée de composés lipidiques, greffés sur le noyau par des liaisons covalentes.

2. Vecteur particulaire selon la revendication 1, caractérisé en ce que le noyau est constitué d'une matrice de polysaccharides ou d'oligosaccharides naturellement ou chimiquement réticulés.

3. Vecteur particulaire selon l'une des revendications 1 et 2, caractérisé en ce que des ligands ioniques sont greffés sur le noyau, et en ce que le noyau présente un caractère ionique.

4. Vecteur selon la revendication 3, caractérisé en ce que les ligands ioniques portent au moins une fonction choisie dans le groupe comprenant : phosphates, sulfates, acides carboxyliques, ammonium quaternaires, amines secondaires, amines primaires.

5. Vecteur particulaire selon l'une des revendications 1 à 4, caractérisé en ce que les composés lipidiques de la couche externe sont des acides gras naturels, fixés au noyau à un taux variable.

6. Vecteur particulaire selon l'une des revendications 1 à 5 , caractérisé en ce qu'il est sous forme lyophilisée.

7. Vecteur particulaire selon l'une des revendications 1 à 5, caractérisé en ce qu'il comporte en outre des molécules d'au moins un médiateur cellulaire à caractère à la fois hydrophobe et ionique, du type agissant par l'intermédiaire d'un récepteur membranaire, et en ce que lesdites molécules sont associées par des interactions hydrophobes à la couche externe de composés lipidiques du vecteur et/ou par des interactions ioniques avec le noyau.

8. Vecteur particulaire selon la revendication 7, caractérisé en ce que les molécules de médiateurs cellulaires sont des polypeptides qui jouent un rôle dans le fonctionnement du système immunitaire.

9. Vecteur particulaire selon l'une des revendications 7 et 8, caractérisé en ce que les molécules de médiateurs cellulaires sont des cytokines choisies dans le groupe comprenant : les interleukines, les interferons, les TNF, le G-CSF, le M-CSF, le GM-CSF et le MIF.

10. Vecteur particulaire selon la revendication 9, caractérisé en ce que la cytokine est l'interleukine 2.

11. Composition pharmaceutique, caractérisée en ce qu'elle comprend des vecteurs particulaires selon l'une des revendications 1 à 10, dans un véhicule pharmaceutiquement acceptable.

12. Procédé d'amplification de l'activité d'un médiateur cellulaire polypeptidique au moins partiellement hydrophobe, caractérisé en ce que
a) on prépare un vecteur particulaire qui comporte de l'intérieur vers l'extérieur
- un noyau constitué d'une matrice de polysaccharides ou d'oligosaccharides naturellement ou chimiquement réticulés,
- une couche externe constituée d'acides gras naturels greffés au noyau par des liaisons covalentes, à un taux variable,
b) de manière facultative, on lyophilise le vecteur obtenu dans un environnement hydrophile.
c) on associe le médiateur cellulaire au vecteur par des interactions au moins partiellement hydrophobes.

13. Procédé d'amplification de l'activité d'un médiateur cellulaire, caractérisé en ce que :
a) on prépare un vecteur particulaire comportant un noyau hydrophile réticulé sur lequel sont greffés des résidus d'acides gras et des ligands ioniques,
b) on lyophilise ledit vecteur particulaire,
c) on met le vecteur lyophilisé en présence d'une quantité limitée d'une solution aqueuse de médiateur cellulaire, de manière à assurer la formation d'agrégats stables de vecteurs chargés en médiateur cellulaire.

## Claims

1. Particulate vector, characterized in that it comprises, from the inside to the outside,
- a nonliquid hydrophilic core,
- an external layer consisting of lipid compounds, grafted on the core by covalent bonds.

2. Particulate vector according to Claim 1, characterized in that the core consists of a matrix of naturally or chemically cross-linked polysaccharides or oligosaccharides.

3. Particulate vector according to either of Claims 1 and 2, characterized in that ionic ligands are grafted onto the core, and in that the core has an ionic character.

4. Vector according to Claim 3, characterized in that the ionic ligands carry at least one functional group chosen from the group comprising: phosphates, sulphates, carboxylic acids, quaternary ammoniums, secondary amines, primary amines.

5. Particulate vector according to one of Claims 1 to 4, characterized in that the lipid compounds of the external layer are natural fatty acids bound to the core at a variable level.

6. Particulate vector according to one of Claims 1 to 5, characterized in that it is in freeze-dried form.

7. Particulate vector according to one of Claims 1 to 5, characterized in that it comprises, in addition, molecules of at least one cellular mediator having both a hydrophobic character and an ionic character, of the type which acts via a membrane receptor, and in that the said molecules are associated by hydrophobic interactions with the external layer of lipid compounds of the vector, and/or by ionic interactions with the core.

8. Particulate vector according to Claim 7, characterized in that the molecules of cellular mediators are polypeptides which play a role in the functioning of the immune system.

9. Particulate vector according to either of Claims 7 and 8, characterized in that the molecules of cellular mediators are cytokines chosen from the group comprising: interleukins, interferons, TNFs, G-CSF, M-CSF, GM-CSF and MIF.

10. Particulate vector according to Claim 9, characterized in that the cytokine is interleukin-2.

11. Pharmaceutical composition, characterized in that it comprises particulate vectors according to one of Claims 1 to 10, in a pharmaceutically acceptable vehicle.

12. Process for enhancing the activity of a polypeptide cellular mediator which is at least partially hydrophobic, characterized in that
a) a particulate vector is prepared which comprises from the inside to the outside
- a core consisting of a matrix of naturally or chemically cross-linked polysaccharides or oligosaccharides,
- an external layer consisting of natural fatty acids grafted onto the core by covalent bonds, at a variable level,
b) optionally, the vector obtained is freeze-dried in a hydrophilic environment,
c) the cellular mediator is associated with the vector via interactions which are at least partially hydrophobic.

13. Process for enhancing the activity of a cellular mediator, characterized in that:
a) a particulate vector is prepared comprising a cross-linked hydrophilic core onto which fatty acid residues and ionic ligands are grafted,
b) the said particulate vector is freeze-dried,
c) the freeze-dried vector is placed in contact with a limited quantity of an aqueous solution of cellular mediator, so as to bring about the formation of stable aggregates of vectors charged with cellular mediator.

## Patentansprüche

1. Teilchen-Träger(Vektor), dadurch gekennzeichnet, daß er, von innen nach außen, enthält:
- einen nicht-flüssigen hydrophilen Kern, und
- eine äußere Schicht, bestehend aus Lipid-Verbindungen, die mittels kovalenter Bindungen auf den Kern aufgepfropft sind.

2. Teilchen-Träger nach Anspruch 1, dadurch gekennzeichnet, daß der Kern besteht aus einer auf natürlichem oder chemischem Wege vernetzten Polysaccharid- oder Oligosaccharid-Matrix.

3. Teilchen-Träger nach einem der Ansprüche 1 und 2, dadurch gekennzeichnet, daß ionische Liganden auf den Kern aufgepfropft sind und daß der Kern einen ionischen Charakter hat.

4. Teilchen-Träger nach Anspruch 3, dadurch gekennzeichnet, daß die ionischen Liganden mindestens eine Funktion tragen, die ausgewählt wird aus der Gruppe, die umfaßt Phosphate, Sulfate, Carbonsäuren, quaternäre Ammonium-Verbindung, sekundäre Amine und primäre Amine.

5. Teilchen-Träger nach einem der Ansprüche 1 bis 4, dadurch gekennzeichnet, daß die Lipid-Verbindungen der äußeren Schicht natürliche Fettsäuren sind, die in einem unterschiedlichen Grad an dem Kern fixiert sind.

6. Teilchen-Träger nach einem der Ansprüche 1 bis 5, dadurch gekennzeichnet, daß er in lyophilisierter Form vorliegt.

7. Teilchen-Träger nach einem der Ansprüche 1 bis 5, dadurch gekennzeichnet, daß er außerdem Moleküle mindestens eines zellulären Mediators mit gleichzeitig hydrophoben und ionischen Eigenschaften eines Typs aufweist, der mittels eines Membranrezeptors wirkt, und daß die genannten Moleküle durch hydrophobe Wechselwirkungen mit der äußeren Schicht aus Lipid-Verbindungen des Trägers (Vektors) und/oder durch ionische Wechselwirkungen mit dem Kern assoziiert sind.

8. Teilchen-Träger nach Anspruch 7, dadurch gekennzeichnet, daß die zellulären Mediator-Moleküle Polypeptide sind, die beim Funktionieren des Immunsystems eine Rolle spielen.

9. Teilchen-Träger nach einem der Ansprüche 7 und 8, dadurch gekennzeichnet, daß die zellulären Mediator-Moleküle Cytokine sind, die ausgewählt werden aus der Gruppe, die umfaßt die Interleukine, die Interferone, die TNF, den G-CSF, den M-CSF, den GM-CSF und den MIF.

10. Teilchen-Träger nach Anspruch 9, dadurch gekennzeichnet, daß es sich bei dem Cytokin um Interleukin 2 handelt.

11. Pharmazeutische Zusammensetzung, dadurch gekennzeichnet, daß sie Teilchen-Träger (Vektoren) nach einem der Ansprüche 1 bis 10 in einem pharmazeutisch akzeptablen Vehikulum enthält.

12. Verfahren zur Verstärkung der Aktivität eines zellulären Polypeptid-Mediators, der mindestens teilweise hydrophob ist, dadurch gekennzeichnet, daß man
a) einen Teilchen-Träger (Vektor) herstellt, der von innen nach außen enthält:
- einen Kern, bestehend aus einer auf natürlichem oder chemischem Wege vernetzten Polysaccharid- oder Oligosacchand-Matrix, und
- eine äußere Schicht, bestehend aus natürlichen Fettsäuren, die durch kovalente Bindungen in variablem Umfang auf den Kern aufgepfropft sind,
b) den erhaltenen Vektor in einer hydrophilen Umgebung gegebenenfalls lyophilisiert und
c) den zellulären Mediator durch mindestens teilweise hydrophobe Wechselwirkungen mit dem Träger (Vektor) assoziiert.

13. Verfahren zur Verstärkung der Aktivität eines zellulären Mediators, dadurch gekennzeichnet, daß man
a) einen Teilchen-Träger (Vektor) herstellt, der einen vernetzten hydrophilen Kern aufweist, auf den Fettsäurereste und ionische Liganden aufgepfropft sind,
b) den genannten Teilchen-Träger (Vektor) lyophilisiert und
c) den lyophilisierten Träger (Vektor) in Gegenwart einer begrenzten Menge einer wäßrigen Lösung des zellulären Mediators in der Weise lyophilisiert, daß die Bildung von stabilen Träger (Vektor)-Aggregaten, die mit zellulärem Mediator gefüllt sind, gewährleistet ist.
